# EUROPÄISCHE PATENTANMELDUNG

(11) **EP 4 517 720 A1**
(43) Veröffentlichungstag der Anmeldung: **05.03.2025**
(21) Anmeldenummer: 23194735.9
(22) Anmeldetag: 31.08.2023
(51) Int. Cl.: G09B 23/28, G16H 50/50

(54) **VERFAHREN ZUR SIMULATION VON PHYSIOLOGISCHEN NEURONALEN SIGNALEN**

(71) Anmelder: Anzinger-Weitmann, Manfred, 4060 Leonding (AT)
(72) Erfinder: Anzinger-Weitmann, Manfred, 4060 Leonding (AT)
(74) Vertreter: SONN Patentanwälte GmbH & Co KG

(57) **Zusammenfassung**

Verfahren zur Simulation von physiologischen neuronalen Signalen, die aus dem lumbalen Rückenmark entstehen, insbesondere an Motoneuronen übertragen werden, wobei ein Simulationsmodell mehrere Populationen von Neuronen umfasst, die untereinander erregend oder inhibierend verbunden sind, wobei im Rahmen der Simulation ein Stimulussignal über eine erste Population von Interneuronen umgeleitet wird, wobei die erste Population keinen von anderen Populationen abhängigen Eingangswert aufweist.

## Beschreibung

Die Erfindung betrifft ein Verfahren zur Simulation von physiologischen neuronalen Signalen, die aus dem lumbalen Rückenmark entstehen, insbesondere an Motoneuronen übertragen werden, wobei ein Simulationsmodell mehrere Populationen von Neuronen umfasst, die untereinander erregend oder inhibierend verbunden sind. Außerdem betrifft die Erfindung ein System zur Datenverarbeitung, umfassend Mittel zur Ausführung des besagten Verfahrens und ein Computerprogrammprodukt, umfassend Befehle, die bei der Ausführung des Programms durch einen Computer diesen veranlassen, das besagte Verfahren auszuführen. Insbesondere betrifft die Erfindung ein Verfahren zur Ermittlung eines elektrischen Signals zur Behandlung einer neurologischen Dysfunktion eines Patienten, wobei das besagte Verfahren zur Simulation von physiologischen neuronalen Signalen eingesetzt wird.

Bereits im Jahr 1911 wurde bei den Experimenten von Thomas Graham Brown [Brown, T. G. (1911). The intrinsic factors in the act of progression in the mammal. Proc. R. Soc. Lond. B, 84(572), 308-319.] erkannt, dass das Basismuster des Gehens vom Spinal Cord erzeugt werden kann, ohne dass eine absteigende Aktivierung vom Cortex erfolgt. Brown definierte ebenfalls 1914 [Brown, T. G. (1914). On the nature of the fundamental activity of the nervous centres; together with an analysis of the conditioning of rhythmic activity in progression, and a theory of the evolution of function in the nervous system. The Journal of physiology, 48(1), 18-46.] eine allgemeine Theorie über die Grundschaltung eines zentralen Mustergenerators und verwendete hier erstmals den Begriff des Half-Centers.

Wilson brachte 1961 [Wilson, D. M. (1961). The central nervous control of flight in a locust. Journal of Experimental Biology, 38(2), 471-490. Walker, M. (2017). Why we sleep: Unlocking the power of sleep and dreams. Simon and Schuster.] in seinen Experimenten den Nachweis eines zentralen Mustergenerators ("Central Pattern Generator" - "CPG"), indem er das Nervensystem der Heuschrecke isolierte und das isolierte Nervensystem in der Lage war, Muster zu produzieren, die dem Flug der Heuschrecke gleich waren. Die Ideen von Graham wurden später auch von Lundberg aufgegriffen [Lundberg, A. (1981). Regulatory Functions of the CNS. Motion and Organization Principles.], in dessen Beschreibung des Beugungsreflexes die spinalen Interneuronen Einfluss nehmen und als eine Grundfunktion zur Steuerung des Bewegungsmusters von Säugetieren dienen.

Später wurde der Nachweis durch Untersuchungen an vielen Wirbeltieren erbracht, unter anderem auch bei der Lamprete, einem Wirbelfisch, den Sten Grillner und Kollegen [Grillner, S., Wallen, P., Brodin, L., & Lansner, A. (1991). Neuronal network generating locomotor behavior in lamprey: circuitry, transmitters, membrane properties, and simulation. Annual review of neuroscience, 14(1), 169-199.] für ihre Experimente und Simulationen als Grundlage nahmen.

Die Modelle von Sten Grillner und Kollegen beschreiben das oszillierende Verhalten eines Agonisten zum Antagonisten, das heißt zwei Motoneuronenpools werden abwechselnd vom Mustergenerator stimuliert. Während der aktiven Zeit des Agonisten wird der Antagonist inhibiert. Zur Aktivierung eines Agonisten wird demnach ein bereits oben erwähntes Half-Center benötigt. Zur Simulation der Lamprete wurden mehrere Half-Center in Reihe geschaltet, welche sich nacheinander aktivieren [Grillner, S. (2006). Biological pattern generation: the cellular and computational logic of networks in motion. Neuron, 52(5), 751-766.; Grillner, S. (2011). Control of locomotion in bipeds, tetrapods, and fish. Comprehensive Physiology, 1179-1236.].

Ein ähnliches Modell verwendeten Rybak und Kollegen [Rybak, I. A., Shevtsova, N. A., Lafreniere-Roula, M., & McCrea, D. A. (2006). Modelling spinal circuitry involved in locomotor pattern generation: insights from deletions during fictive locomotion. The Journal of physiology, 577(2), 617-639.; Rybak, I. A., Dougherty, K. J., & Shevtsova, N. A. (2015). Organization of the mammalian locomotor CPG: review of computational model and circuit architectures based on genetically identified spinal interneurons. eNeuro, 2(5), ENEURO-0069.; Danner, S. M., Shevtsova, N. A., Frigon, A., & Rybak, I. A. (2017). Computational modeling of spinal circuits controlling limb coordination and gaits in quadrupeds. Elife, 6, e31050.; Shevtsova, N. A., Hamade, K., Chakrabarty, S., Markin, S. N., Prilutsky, B. I., & Rybak, I. A. (2016). Modeling the Organization of Spinal Cord Neural Circuits Controlling Two-Joint Muscles. In Neuromechanical Modeling of Posture and Locomotion (pp. 121-162). Springer, New York, NY.] zur Modellierung eines solchen Half-Centers. Dieses Modell leitet sich von Untersuchungen an der Muskulatur der Atmung ab. Bei diesen Untersuchungen wurden in den zugehörigen Neuronenpools Ionenkanäle identifiziert, welche sehr hohe Zeitkonstanten aufweisen - die persistenten Natriumkanäle NaP, die zur Rhythmusgenerierung herangezogen werden [Paul, J. R., DeWoskin, D., McMeekin, L. J., Cowell, R. M., Forger, D. B., & Gamble, K. L. (2016). Regulation of persistent sodium currents by glycogen synthase kinase 3 encodes daily rhythms of neuronal excitability. Nature communications, 7, 13470.].

Es ist eine Aufgabe der gegenständlichen Erfindung, unter Verbesserung der obigen Modelle eine möglichst realistische (und genaue) Simulation der neuronalen Abläufe bereitzustellen, die für Muskelbewegungen von Menschen und Tieren relevant sind. Die Simulation hat damit den einzigen (vernünftig anzunehmenden) Anwendungszweck, die einen Patienten beobachteten und gemessenen physiologischen neuronalen Signale nachzubilden, die für eine Bewegung ausschlaggebend sind, und die körpereigenen Signale allenfalls durch elektrische Stimulation zu ergänzen bzw. die Auswirkungen solcher Ergänzungen vorab zu überprüfen, deren Signale und Muster anhand der Simulation ermittelt werden können. Dadurch soll letztendlich beispielsweise die Behandlung einer motorischen Dysfunktion ermöglicht oder zumindest verbessert werden.

Diese Aufgabe wird durch die in den Ansprüchen definierte Erfindung gelöst.

Es hat sich herausgestellt, dass eine wesentliche Komponente einer realistischen Simulation darin besteht, dass im Rahmen der Simulation ein Stimulussignal über eine erste Population von Interneuronen umgeleitet wird, wobei die erste Population keinen von anderen Populationen abhängigen Eingangswert aufweist.

Ein Verfahren zur Simulation von physiologischen neuronalen Signalen wie oben beschrieben und im Folgenden durch speziellere Beispiele und optionale Varianten weiter erläutert kann insbesondere im Rahmen eines Verfahrens zur Ermittlung eines elektrischen Signals zur Behandlung einer neurologischen Dysfunktion eines Patienten eingesetzt werden. Dabei wird ausgehend von gemessenen physiologischen neuronalen Signalen des Patienten das Simulationsmodell zur Nachbildung dieser physiologischen neuronalen Signale angepasst. Anhand des angepassten Simulationsmodells wird ein externes elektrisches Signal simuliert und es werden die Parameter des elektrischen Signals derart angepasst, dass die nachgebildeten physiologischen neuronalen Signale einem Signalverlauf ohne Dysfunktion angenähert werden. Die Anpassung kann beispielsweise in einer Veränderung eines statischen elektrischen Potenzials bestehen. Alternativ kann auch die Frequenz und Amplitude eines periodischen Stimulussignals verändert werden. Der Vergleich mit dem Signalverlauf ohne Dysfunktion kann dabei einen Toleranzbereich aufweisen. Es kann sich beispielsweise um ein nur qualitativ Vergleich handeln oder um einen Abstand zu einem idealen Verlauf, wobei eine Anpassung bereits akzeptiert wird, wenn der Abstand zwischen dem durch die Simulation erzielten Ausgangssignal dem ursprünglichen Ausgangssignal größer ist als der Abstand zwischen dem durch die Simulation erzielten Ausgangssignal und einem idealen Ausgangssignal.

Das obige Verfahren zur Ermittlung eines elektrischen Signals kann beispielsweise zur Behandlung einer neurologischen Dysfunktion eines Patienten im Bereich des Bewegungsapparats, insbesondere im Bereich des lumbalen Rückenmarks, verwendet werden. Dazu kann etwa das ermittelte elektrisches Signal über Elektroden am Körper, z.B. einzelnen Gliedmaßen, des Patienten angelegt werden.

Im Folgenden werden einige optionale Merkmale erläutert, die zu einer realistischen Simulation zusätzlich beitragen.

Die erste Population kann inhibierend auf eine zweite Population von Neuronen wirken, wobei die zweite Population einem zentralen Mustergenerator angehört, dem zumindest eine dritte Population von Neuronen im Simulationsmodell angehört. Der zentrale Mustergenerator umfasst somit mindestens zwei - simulierte - Populationen von Neuronen, die zweite und dritte Population. Die verschiedenen Populationen bilden zusammen ein schwingendes System, dessen dynamisches Verhalten letztlich für den Verlauf des erzeugten Musters und damit des erzeugten Ausgangssignal prägend ist.

Beispielsweise kann die zweite Population erregend auf die dritte Population wirken und die dritte Population umgekehrt mit einer Verzögerung hemmend auf die zweite Population wirken.

Die besagte Verzögerung kann beispielsweise im einfachsten Fall durch eine Zeitkonstante (T) definiert und parametrisiert werden, wobei die Zeitkonstante (T) zwischen 0,1 und 30 Millisekunden liegen kann, insbesondere zwischen 0,1 und 20 Millisekunden. Die Zeitkonstante kann beispielsweise als Konstante einer exponentiellen Abnahme der Aktivierung einer Population der Simulation berücksichtigt werden.

Die dem zentralen Mustergenerator angehörenden Populationen können so gewählt sein, dass sie sich vorwiegend erregen. D. h. zwischen den Populationen bestehen in diesem Fall gemessen an der Anzahl überwiegend erregende Verbindungen, mehr als hemmende Verbindungen.

Dem zentralen Mustergenerator kann zumindest eine vierte Population von Neuronen im Simulationsmodell angehören, wobei vorzugsweise die vierte Population mit der zweiten Population gegenseitig erregend wirkt und die vierte Population auf die dritte Population erregend wirkt und umgekehrt von der dritten Population mit einer Verzögerung gehemmt wird. Diese gegenseitigen Wirkungen zwischen den Populationen, sei es erregend oder hemmend, können jeweils individuell mit Verzögerungen verbunden sein, die jeweils mit einer eigenen Zeitkonstante parametrisiert sind. Die vierte Population verbessert die Selbsterregung innerhalb des Mustergenerators. Die Inhibierung (oder Hemmung) sowohl der zweiten als auch der vierten Population durch die dritte Population wird durch das Stimulussignal durchbrochen, das beispielsweise ein konstanter Stimulus sein kann. Dadurch wird das System, d. h. der zentrale Mustergenerator, ausreichend rasch erregt, um eine Oszillationen auszulösen.

Gemäß einer weiteren Ausführungsform kann dem zentralen Mustergenerator zumindest eine fünfte Population von Neuronen im Simulationsmodell angehören, wobei die fünfte Population eine selbst erregende Population ist, wobei die fünfte Population vorzugsweise erregend auf die zweite Population wirkt. Dadurch kann die hemmende Wirkung der ersten Population auf die zweite Population periodisch kompensiert werden. Daraus resultierend wird eine annähernd lineare Reaktion des Mustergenerators auf das Stimulussignal erreicht. Im Einzelnen kann bei dieser Ausführungsform ein (zumindest in einem Arbeitsbereich) proportionaler Zusammenhang zwischen der Höhe eines konstanten Stimulussignals und der Ausgangsfrequenz erzielt werden.

Bei sämtlichen obigen Varianten kann ein simuliertes physiologisches neuronales Signal durch einen Ausgangswert des zentralen Mustergenerators gebildet sein, wobei dieser Ausgangswert vorzugsweise mit einem gemessenen physiologischen neuronalen Signal verglichen wird, um das Simulationsmodell anzupassen. Dabei wird insbesondere die Amplitude und Frequenz des Ausgangssignals ausgewertet. Je nach gewünschter bzw. erwarteter neurologischer Reaktion kann das gemessene physiologische neuronale Signal zu (wenn eigentlich kein Signal an der betreffenden Stelle ausgelöst werden sollte) oder zu gering (wenn das gemessene Signal im Vergleich zu anderen gemessenen Signalen stärker hervortreten sollte) sein. Es kann daher zunächst durch Änderung der Parameter des Simulationsmodells und des Stimulussignals nach einer Konfiguration der Simulation gesucht werden, die das gemessene Verhalten reproduziert. Anschließend kann durch eine Veränderung des Stimulus versucht werden, das Ausgangssignal der Simulation einem gewünschten Ausgangssignal anzunähern. Die Parameter dieser Veränderung geben Aufschluss über eine mögliche Erleichterung der beobachteten Dysfunktion.

Beispielsweise kann bei der obigen Konfiguration des Mustergenerators ein Ausgangswert der vierten Population als Ausgangswert des zentralen Mustergenerator verwendet werden.

Um darüber hinaus die Amplitude des Ausgangssignals besser regeln zu können, kann alternativ vorgesehen sein, dass dem zentralen Mustergenerator zumindest eine sechste Population von Neuronen im Simulationsmodell angehört, wobei die sechste Population von der vierten Population erregt wird und wobei ein Ausgangswert der sechsten Population als Ausgangswert des zentralen Mustergenerator verwendet wird. Zur Regelung des Ausgangssignals kann dabei ein weiterer externer Stimulus an der sechsten Population simuliert werden.

Anstelle des direkten Stimulus an der sechsten Population kann alternativ dem zentralen Mustergenerator zumindest eine siebte Population von Neuronen Simulationsmodell angehören, wobei die siebte Population hemmend auf die sechste Population wirkt. Ähnlich dem Stimulus auf die erste Population, die die zweite Population hemmend kann der Stimulus auf siebte Population letztlich hemmend auf die sechste Population wirken, mit dem für Wirkungen zwischen den Populationen für die Simulation angewendeten Zeitverhalten.

In einer weiteren Verfeinerung der Simulation können langsamere Effekte auf die Dynamik des Systems beispielsweise dadurch berücksichtigt werden, dass die Wechselwirkung zwischen den Populationen durch Moderationsparameter beeinflusst wird, wobei jeder Population ein Moderationsparameter jeweils zugeordnet ist, wobei die Moderationsparameter ihrerseits einer zeitlichen Entwicklung folgen, die von der Erregung der jeweiligen Population abhängig ist. Auf diese Weise kann die Wirkung des in der Natur beobachteten glialen Systems auf die Wechselwirkungen zwischen den Neuronen in vereinfachter und effektiver Weise berücksichtigt werden.

Die Erfindung wird nachfolgend anhand von besonders bevorzugten Ausführungsbeispielen, auf die sie jedoch nicht beschränkt sein soll, und unter Bezugnahme auf die Zeichnungen weiter erläutert. Dabei zeigen im Einzelnen:
Fig. 1 zeigt eine graphische Darstellung eines Metamodells;
Fig. 2 zeigt beispielhafte Parameter im Metamodell zur Definition eines externen Störsignals;
Fig. 3 zeigt einen Mustergenerator ("Half-Center") nach Rybak;
Fig. 4 zeigt eine erste Ausführungsform eines Mustergenerators der hier gegenständlichen Art und des damit erzielten Ausgangssignals;
Fig. 5 zeigt die im Beispiel gemäß Fig. 4 verwendet Übertragungsfunktion;
Fig. 6 zeigt eine zweite Ausführungsform eines Mustergenerators der hier gegenständlichen Art und des damit erzielten Ausgangssignals;
Fig. 7 zeigt eine dritte Ausführungsform eines Mustergenerators der hier gegenständlichen Art und des damit erzielten Ausgangssignals;
Fig. 8 zeigt ein vollständiges Simulationsmodell auf Basis des in Fig. 4 dargestellten Mustergenerators mit Agonist und Antagonist;
Fig. 9 zeigt das Ergebnis der Simulation in Form der generierten Ausgangssignale des in Fig. 8 gezeigten Modells;
Fig. 10 zeigt eine vierte Ausführungsform des Mustergeneerators und
Fig. 11 zeigt die entsprechenden Ausgangssignale.

Zur Bewerkstelligung der Aufgabe, ein flexibles Modell beispielsweise des Rückenmarks und dessen verschieden Eigenschaften zu simulieren, ist es nützlich, eine möglichst flexible, leistungsfähige Simulationsumgebung in Form von Standard-Software zu verwenden. Die gewählte Standard-Software soll insbesondere dem Modell zugrunde liegende Differenzialgleichungen (numerisch) lösen und dadurch die Simulation ausführen können. Für die folgenden Ausführungsbeispiele wurde die Simulationsumgebung in dem Software-Produkt "MATLAB" der Firma MathWorks in Form einer eigens dafür entwickelten und allgemein verfügbaren Toolbox eingesetzt [Anzinger, M. (2005). Toolbox zur Simulation inhomogener Populationen. Anwendung im Bereich Neuronenpopulationen. Diplomarbeit an der Medizinischen Universität Wien, 1-83.]. Die im Folgenden referenzierten Komponenten diese Toolbox bilden Teil der gegenständlichen Offenbarung.

Die in allen Figuren verwendete grafische Notation des Simulationsmodells soll zunächst anhand eines einfachen Beispiels und dem korrespondierenden Programmcode gezeigt werden.

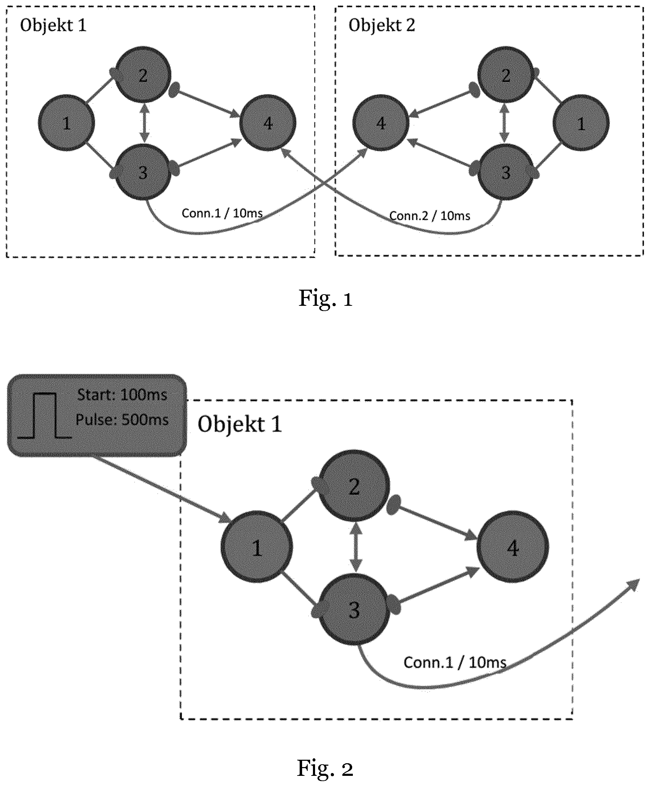

In Fig. 1 wird ein Beispiel der graphischen Darstellung eines Metamodells gezeigt. Das Modell enthält zwei Objekte mit je vier Populationen. Jedes Objekt hält im Hintergrund eine 4×4 Matrix, die die internen Verbindungen wiedergibt und einen Vektor gleicher Länge für die innere Zeitkonstante. Eine Ellipse definiert eine negative, ein Pfeil eine positive synaptische Verbindung. Die geschwungenen Linien Conn.1 und Conn. 2 definieren eine singuläre synaptische Verbindung mit einer Latenzzeit von 10 ms (Millisekunden).

Der korrespondierende Programmcode lautet, aufbauend auf der oben referenzierten Toolbox:

```
       % struct net
       Metamodel=createModel() ;
       % necessary time constants
       tau_Obj1 = [20, 10, 20, 10];
       tau_Obj2 = [20, 10, 20, 10];
       % own weight matrix
       wMat = [0 0 0 0;
                -1 0 1 -1;
                -1 1 0 -1;
                  0 1 1 0];
       % Füge Objekt 1 und 2 zu Metamodel mit Matrix und
       Zeitkonstanten;
       [Metamodel, Obj1] = addPO(Metamodel, tau_Obj1, 'wMatrix',
       wMat);
       [Metamodel, Obj2] = addPO(Metamodel, tau_Obj2, 'wMatrix',
       wMat);
       % Füge Connection_1 und 2 zu Metamodel mit Gewicht 1 und
       Latenzzeit 10ms
       Metamodel = connectPO(Metamodel,
       'src',Obj1.node{3}, 'dest',Obj2.node{4}, 'weight', 1,
       'delay', 10);
       Metamodel = connectPO(Metamodel,
       'src',Obj2.node{3}, 'dest',Obj1.node{4}, 'weight', 1,
       'delay', 10);
```

Die Funktionen addPO und connnectPO befüllen das Metamodell mit den Informationen.

Der Stimulus ist ein Teil des Metamodells. Er definiert die externen Störgrößen im System. Im graphischen Metamodell können wir diesen leicht als einen Signalgenerator darstellen. Dieser muss dann für die gesamte zu simulierende Zeitperiode zeitrichtig das Störsignal zur Verfügung stellen. Dazu verwendet dieser Informationen über die Form des Signals, die Zielpopulation, die zu simulierende Zeitperiode und Information über die Zeitdiskretisierung des Solver.

Die Diskretisierung wird hier bei konstanter Schrittweite berechnet. Das Störsignal kann daher zu Beginn der Simulation vorberechnet und dem Modell während der Laufzeit zeitrichtig zur Verfügung gestellt werden. In Fig. 2 sind beispielhafte Parameter im Metamodell zur Definition eines externen Störsignals dargestellt. Stim.1-n gibt die jeweilige Störgröße an. Der Parameter Stimuli Vector stellt die Funktion der Störgröße mit der korrekten Zeitdiskretisierung zur Laufzeit dem Modell zur Verfügung. Der Parameter Time gibt an, zu welchem Zeitpunkt die Störgröße dem Modell beaufschlagt wird. Der Parameter Node bezeichnet die Zielpopulation im Modell. Der korrespondierende Programmcode lautet:

```
       % generiere ein Rechteck mit der Pulslänge von 600ms
       % und einer Amplitude von 0,65.
       S1=ones(1,600/dt)*0.65;
       % füge den Stimulus S1 zum Metamodel
       % zum Zeitpunkt 100ms hinzu.
       Metamodel = addStimulus(net, Pendel.node{1}, S1, 100);
```

Der Integrator bildet das Herz der Simulation. Die relevanten Informationen für das simulierte neuronale System werden hier übergeben. Die Parameter des hier eingesetzten Integrators sind die zu simulierende Zeitspanne tspan und zugehörige Schrittweite dt, das Metamodell metaModel, das verwendete Differentialgleichungssystem für eine einzelne homogene Population welches im Parameter solver hinterlegt ist und zuletzt noch die Übertragungsfunktion welche durch den Parameter sigm festgelegt wird.

```
       dt=0.1; % fixe Integratorschrittweite in ms
       tspan=100; % zu Berechnende Zeitspanne in ms
       result = runModel(Metamodel, dt, tspan, 'solver', 'S2',
       'sigm', @sigm);
```

Für den Solver selbst wird hier das Runge-Kutta Verfahren 4. Ordnung verwendet.

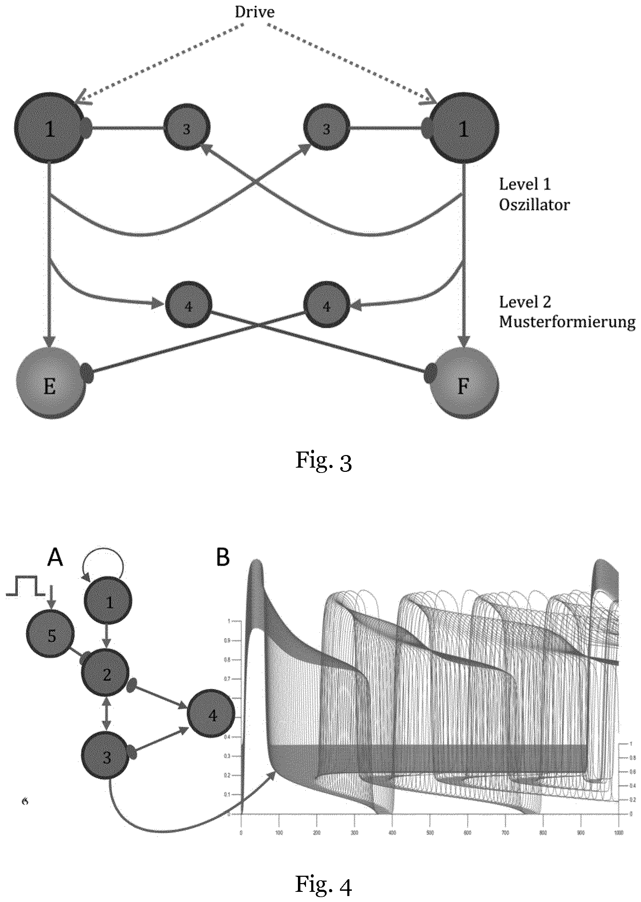

Zur Darstellung des Standes der Technik und zum Vergleich zeigt Fig. 3 ein Mustergenerator ("Half-Center") sind nach Rybak [McCrea, D. A., & Rybak, I. A. (2008). Organization of mammalian locomotor rhythm and pattern generation. Brain research reviews, 57(1), 134-146.] in der hier verwendeten grafischen Notation. Rote Kreise sind erregende Interneuronen (Populationen), blaue Kreise sind inhibierende Interneuronen. Kreis E stellt die Extensor Motoneuronen und Kreis F die Flexor Motoneuronen dar. Ein Half-Center nach Rybak besitzt die Fähigkeit einer alternierenden Oszillation von Flexor und Extensor. Eine Oszillation eines einzelnen Motoneuronenpool ist nicht möglich. Die Neuronen einer Population werden nach Hodgkin-Huxley Style modelliert und bestehen aus ca. 20 - 100 einzelmodellierten Neuronen.

Als Beispiel aus der funktionalen Elektrostimulation ist hier zu erwähnen, dass durch einen konstanten Stimulus (konstante Frequenz und konstante Amplitude) an den afferenten Signalen des lumbalen Spinal-Cord, das Gehmuster eines Patienten mit einer kompletten Querschnittslähmung ausgelöst werden konnte. Allerdings ist es durch die Anbringung eines konstanten Stimulus bei der funktionalen Elektrostimulation viel häufiger der Fall, unerwünschte Bewegungsmuster zu hemmen, als diese zu aktivieren.

In den Modellen von Rybak und Kollegen wird ein konstanter Stimulus an beiden Motorunits des Half-Centers angebracht, welcher die nötige Erregung zur Erzeugung einer Oszillation liefert. Wird dieser Stimulus in seiner Amplitude erhöht, erhöht sich auch die Frequenz der Oszillation. Werden an beide Motorunits Stimuli mit verschiedener Stärke angebracht, verschiebt sich dem Verhältnis entsprechend, die aktive Zeit von Agonist zu Antagonist und vice versa - Fig. 3.

Fig. 4 zeigt eine Simulation und insbesondere einen Mustergenerator der hier gegenständlichen Art. Im linken Teil ist der Mustergenerator gemäß der oben angeführten grafischen Notation dargestellt; im rechten Teil dessen Reaktion auf die Änderung des Stimulussignals. Dazu wird ein Stimulus an Population 5 (die erste Population) angelegt, welcher über einen bestimmten Wert variiert wird. Jede Änderung wird in einem Wasserfallplot dargestellt. Der Stimulus wird von 0 - +1 variiert. B: Eine starke Linearisierung des Systems über den Stimulus ist zu sehen die sich durch die Einführung des zusätzlichen Interneurons (erste Population) ergibt. Die Übertragungskennlinie der sigmoidalen Funktion bildet sich über die Impulsbreite ab. Der Impuls wird ab einer Stimulusstärke von ca. 0.6 vollständig inhibiert.

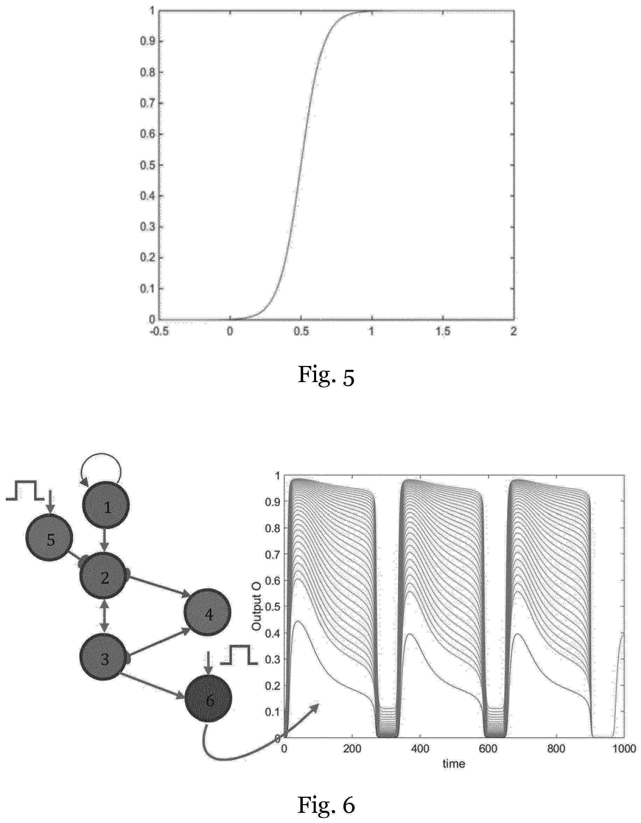

Die sigmoidale Funktion bildet sich von einem Stimuluswert von 0 - 0,6 ab. Dies liegt vorwiegend an der verwendeten Übertragungsfunktion in Fig. 5. Diese gibt bei einem Input von 0,6 bereits den Output von 0,9 aus. D.h. um den zu regelnden Bereich auf einen Input von 0,1 - 0,9 zu dehnen, müsste die Übertragungsfunktion einen flacheren Verlauf aufweisen. Wird die Übertragungsfunktion geändert, müssen jedoch auch die Gewichte im Modell angepasst werden.

Der korrespondierende Programmcode dieser Simulation lautet:

```
       % struct net
       net=createModel();
       % necessary time constants for models 1 and 2.
       ptau=[1, 20, 3, 30, 3]; % für Modell S2
       % ptau=[0.22, 0.72]; % für Modell S1
       % own weight matrix
       wMat=[ 3.3 0 0 0 0;
                 1 0 2.2 -1.8 -1;
                 0 1.8 0 -1.2 0;
                 0 1 1 0 0
                 00000];
       y=[0.2, 0, 0, 0, 0];
       [net P]=addPO(net, ptau, 'wMatrix', wMat, 'inity1', y);
       % create stimuli
       dt=0.1; % stepwidth of the integrator
       tspan=1000; % total time span
       stimStart=100;
       tStim=tspan-stimStart;
       S2=ones(1,tStim/dt)*0.; % rectangle
       figure(1) ;
       elf;
       while S2<0.99
            net.cntStimulus=0;
            net = addStimulus(net, P.node{5}, S2, 10);
            % start simulation
            R = runModel (net, dt, tspan, 'solver', 'S2', 'sigm',
       @sigm2);
            hold on;
            x=R.PO(1).y1(3,:);
            y=R.t;
            z=ones(1,length(R.t))*S2(1);
            plot3 (y, z,x);
            S2=S2+0.01;
       end;
       view(0,20) ;
```

Gemäß einem weiteren Ausführungsbeispiel wird eine weitere Population in unser Modell eingeführt, welche nur zur Steuerung der Amplitude Verwendung findet. Diese Population wird mit einem Gewicht <1 verbunden, um die resultierende Amplitude zu verringern. Mit einem zusätzlichen Stimulus wird die Höhe anschließend geregelt.

In unser Modell wurde die Population 6 eingefügt, welche mit einem Gewicht von 0,5 von Population 3 erregt wird. In Fig. 6 ist nun der Output von Population 6 ersichtlich. Die blaue Linie zeigt den Output ohne zusätzlichen Stimulus. Anschließend wurde ein konstanter Stimulus über die gesamte Zeit beginnend mit einer Stärke von 0,05 bis 0,35 in 0,01 Schritten an Population 6 angebracht. Man kann sehen, dass die Amplitude mit Zunahme des Stimulus verstärkt wurde. Eine geringfüge Anhebung des Bias ist zu sehen. Würde man jedoch den Stimulus weiter erhöhen, würde sich der Bias entsprechend erhöhen. Die Amplitude ist somit hier nur innerhalb eines kleinen Bereichs des Inputsignals regelbar.

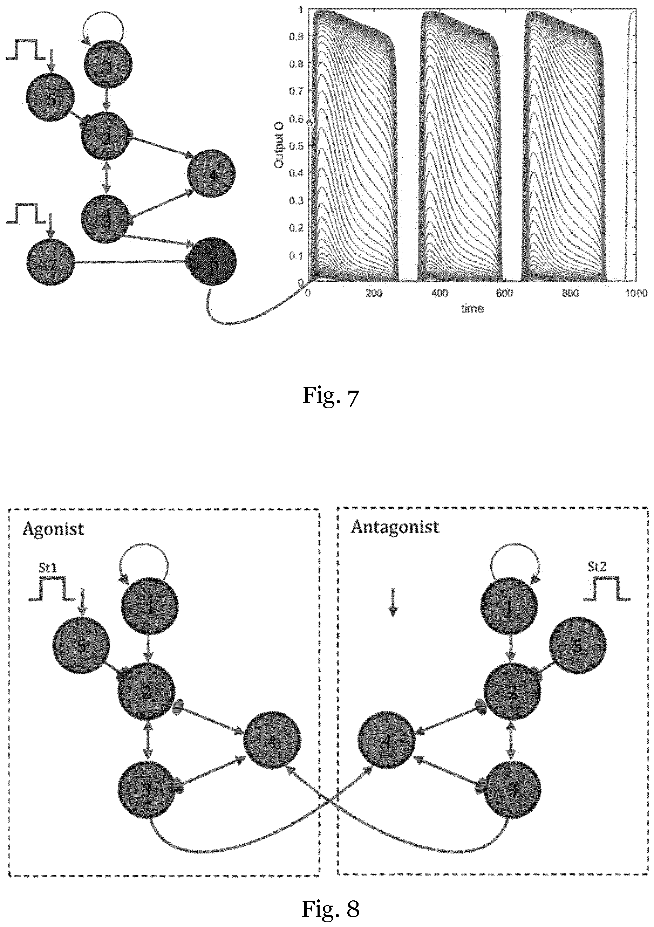

Um dieses Problem zu umgehen wird wieder, anstatt dem Output einen Stimulus zu geben, angenommen, dass dieser bereits zu Beginn eine maximale Amplitude besitzt und durch ein inhibierendes Interneuron in seiner Amplitude geregelt wird. Das entspricht der in Fig. 7 dargestellten dritten Ausführungsform. Dabei ist ersichtlich, dass die Amplitude über den gesamten Bereich geregelt werden kann. Der Bias Fehler wie zuvor tritt nicht mehr in Erscheinung.

Zur Beschreibung eines Half-Centers verwendet man einen Agonisten sowie den zugehörigen Antagonisten. Die klassische Medizin geht davon aus, dass die Motoneuronen des Antagonisten inhibiert werden, wenn dieser durch Aktivierung des Agonisten gedehnt wird. Tatsächlich wird bei jeder Bewegung auch der Gegenspieler aktiviert, um das jeweilige Gelenk zu stabilisieren. Das ist auch in den jeweiligen Muskelableitungen von nicht beeinträchtigten Personen gut zu sehen und wichtig, um die jeweiligen Aktvierungsmuster richtig zu interpretieren.

Um nun das Spiel von Agonist und Antagonist zu modellieren, nehmen wir das Modell eines Mustergenerators wie in Fig. 4 für jede Muskelgruppe. Ist die erste Muskelgruppe - wir nennen sie Agonist - aktiv, so inhibiert diese über eine laterale Verbindung die zweite Muskelgruppe - wir nennen sie Antagonist (Fig. 8). Hier ist der entsprechende Programmcode der Simulation:

```
       % struct net
       net=createModel();
       % necessary time constants for models 1 and 2.
       ptau=[1, 20, 3, 30, 3]; % für Modell S2
       % own weight matrix
       wMat=[ 3.3 0 0 0 0;
                 1 0 2.2 -1.8 -1;
                 0 1.8 0 -1.2 0;
                 0 1 1 0 0;
                 0 0 0 0 0];
       yAg=[0.2, 0.1, 0, 0, 0];
       yAn=[0.2, 0., 0, 0.2, 0];
       % Collect Populatios to the net
       [net Agon]=addPO(net, ptau, 'wMatrix', wMat, 'inity1', yAg);
       [net Antagon]=addPO(net, ptau, 'wMatrix', wMat, 'inity1',
       yAn) ;
       %lateral connections - Afferenzen
       net = connectPO(net,
       'src',Agon.node{3},'dest',Antagon.node{4},'weight',0.9);
       net = connectPO(net,
       'src',Antagon.node{3}, 'dest',Agon.node{4}, 'weight',0.9);
       % create stimuli
       dt=0.1; % stepwidth of the integrator
       tspan=1000; % total time span
       stimStart=100;
       tStim=tspan-stimStart;
       S1=ones(1,1/dt); % Dirac Impuls (with 1 ms length)
       S2=ones(1,tStim/dt)*0.; % rectangle
       S4=ones(1,tStim/dt)*0.; % rectangle
       figure(1); elf;
       while S2<0.99
            net.cntStimulus=0;
            net = addStimulus(net, Agon.node{5}, S2, 0);
            net = addStimulus(net, Antagon.node{5}, S2, 0);
            % start simulation
            R = runModel(net, dt, tspan, 'solver', 'S2', 'sigm',
       @sigm2);
            hold on;
            x=R.PO(1).y1(3,:);
            y=R.t;
            z=ones(1,length(R.t))*S2(1);
            plot3(y,z,x,'b');
            x=R.PO(2).y1(3,:);
            y=R.t;
            z=ones(1,length(R.t))*S2(1);
            plot3(y,z,x,'r');
            S2=S2+0.01;
       end;
```

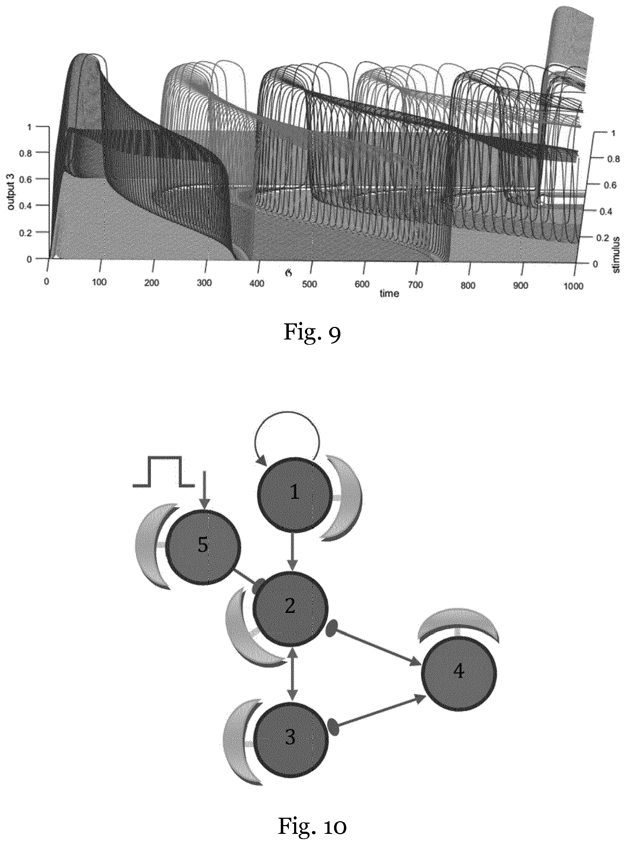

Solange der Agonist in Population 3 aktiv ist, inhibiert diese über die laterale Verbindung nach Population 4 die gesamte Muskelgruppe des Antagonisten. Ist die aktive Zeit vorbei und wird der Agonist ausreichend inhibiert, erhält der Antagonist die Überhand und wird aktiv. Über die laterale Verbindung nach Population 4 des Agonisten wird dieser dann inhibiert. Die Dauer des Pulses wird für jede Gruppe jeweils über Stimulus St1 bzw. über Stimulus St2 unabhängig geregelt - das Ergebnis der Simulation in Form der generierten Ausgangssignale ist in Fig. 9 gezeigt. Blaue Linien sind der Output des Agonisten an Population 3, rote Linien sind der Output des Antagonisten an Population 3. An Population 4 beider Gruppen wurde ein Stimulus angebracht welcher von 0 - 1 variiert wurde. Die Frequenz erhöht sich mit Zunahme des Stimulus. Während einer der beiden Gruppen aktiv ist, wird die kontralaterale Gruppe inhibiert. Die beiden Populationen wurden übereinander geplottet.

Um von außen auf das Verhalten von Aktivität und Ruhe einzugreifen, müssen die afferenten Signale einer Gruppe berücksichtigt werden. Hier wäre vor allem wichtig zu erkennen, wenn eine Bewegung, z.B. beim Gangmuster, zu Ende ist, in dem ein afferentes Signal beim Kontaktieren des Bodens an den jeweiligen CPG gesendet wird [Prochazka, A., Gosgnach, S., Capaday, C., & Geyer, H. (2017). Neuromuscular models for locomotion. In Bioinspired Legged Locomotion (pp. 401-453). Butterworth-Heinemann.; Moraud, E. M., Zitzewitz, J., Miehlbradt, J., Wurth, S., Formento, E., DiGiovanna, J., ... & Micera, S. (2018). Closed-loop control of trunk posture improves locomotion through the regulation of leg proprioceptive feedback after spinal cord injury. Scientific reports, 8(1), 76.]. Dieses müsste eine zusätzliche Inhibierung des CPG bewirken und die Aktivität vorzeitig beenden.

Im vierten Ausführungsbeispiel wurde das Modell (siehe Fig. 10) dahingehend erweitert, dass jede Population ein zugehöriges gliales System erhält (gelber Halbmond). Durch die modellierte Selbsterregung an Population 1 kann sich das Modell zuvor (ohne gliales System) ohne Zutun eines externen Stimulus auf ein bestimmtes Maximum erregen. Die Konnektivität zwischen den Populationen ist aufgrund des glialen Systems dynamisch. Der Mustergenerator ist selbsterregt, ein Stimulus an Population 5 ermöglicht es, diesen zu steuern. Der zugehörigen Programmcode einer entsprechenden Simulation ist beispielsweise:

```
       % struct net
       net=createModel();
       % necessary time constants for models 1 and 2.
       ptau=[20, 10, 3, 30, 30]; % für Modell S2
       alpha =[0.001 0.001 0.001 0.001 0.0001];
       beta =[0.03 0.03 0.02 0.03 0.03];
       % own weight matrix
       wMat=[ 0.9 0 0 0 0; % P1
                 1 0 2.5 -1.7 -0.8; % P2
                 0 1.8 0 -1.2 -0.; % P3
                 0 1 1 0 0 % P4
                 0 0 0 0 0]; % P5
       y=[0.7, 0.7, 0.3, 0.01, 0.3];
       [net, P]=addPO(net, ptau, 'wMatrix', wMat, 'Alpha', alpha,
       'Beta', beta, 'inity1', y);
       % create stimuli
       dt=0.05; % stepwidth of the integrator
       tspan=3000; % total time span
       S4=ones(1,50/dt)*3; % rectangle
       net = addStimulus(net, P.node{1}, S4, 10);
       % start simulation
       R = runModel(net, dt, tspan, 'solver', 'S2', 'sigm',
       @sigm2Ca);
```

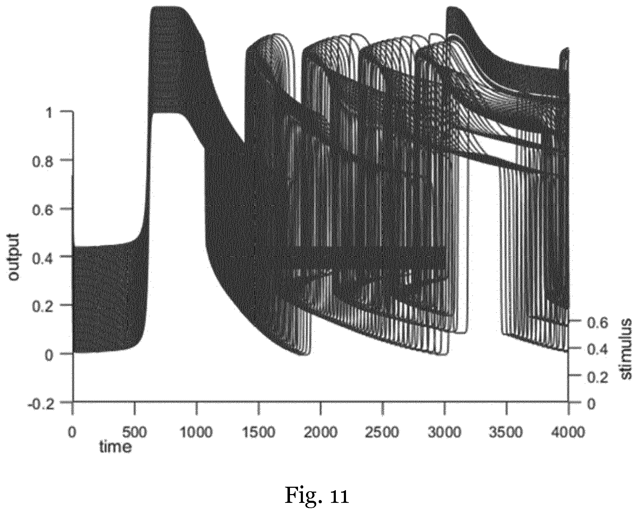

Fig. 11 zeigt, dass sich das System noch regeln lässt. Dazu werden wir wieder einen Stimulus an Population 5 anbringen und diesen über die Amplitude variieren. Fig. 11 zeigt genauer das Ausgangssignal von Population 3 - der Stimulus an Population 5 wurde von 0.01 bis 0.9 variiert.

Der Mustergenerator verhält sich wie erwartet ähnlich wie zuvor. Die sigmoidale Übertragungsfunktion ist allerdings im Wasserfalldiagramm nicht mehr ersichtlich. Ein linearer Regelbereich ist bei einem Stimulus von 0.1 bis ca. 0.6 gegeben.

Die vorliegende Offenbarung enthält Teile der Dissertation "Modellierung des lumbalen Rückenmarks zur Erklärung funktionaler Abweichungen des Bewegungsmusters bei Querschnittpatienten" von Manfred Anzinger-Weitmann, TU Wien, die am Tag der Einreichung dieser Anmeldung veröffentlicht wird. Der für das Verständnis und die Nachvollziehbarkeit der Erfindung erforderlichen Teile wurden hier wiedergegeben. Sollten weitere Teile für diese Anmeldung nützlich sein, sollen diese mit diesem Verweis als Teil dieser Offenbarung gelten und bei Bedarf übernommen werden.

## Patentansprüche

1. Verfahren zur Simulation von physiologischen neuronalen Signalen, die aus dem lumbalen Rückenmark entstehen, insbesondere an Motoneuronen übertragen werden, wobei ein Simulationsmodell mehrere Populationen von Neuronen umfasst, die untereinander erregend oder inhibierend verbunden sind,
**dadurch gekennzeichnet, dass**
im Rahmen der Simulation ein Stimulussignal über eine erste Population von Interneuronen umgeleitet wird, wobei die erste Population keinen von anderen Populationen abhängigen Eingangswert aufweist.

2. Verfahren nach Anspruch 1, **dadurch gekennzeichnet, dass** die erste Population inhibierend auf eine zweite Population von Neuronen wirkt, wobei die zweite Population einem zentralen Mustergenerator angehört, dem zumindest eine dritte Population von Neuronen im Simulationsmodell angehört.

3. Verfahren nach Anspruch 2, **dadurch gekennzeichnet, dass** die zweite Population erregend auf die dritte Population wirkt und die dritte Population umgekehrt mit einer Verzögerung hemmend auf die zweite Population wirkt.

4. Verfahren nach Anspruch 3, **dadurch gekennzeichnet, dass** die Verzögerung durch eine Zeitkonstante (T) definiert wird, wobei die Zeitkonstante (T) zwischen 0,1 und 30 Millisekunden liegt, insbesondere zwischen 0,1 und 20 Millisekunden.

5. Verfahren nach einem der Ansprüche 2 bis 4, **dadurch gekennzeichnet, dass** die dem zentralen Mustergenerator angehörenden Populationen so gewählt sind, dass sie sich vorwiegend erregend.

6. Verfahren nach einem der Ansprüche 2 bis 5, **dadurch gekennzeichnet, dass** dem zentralen Mustergenerator zumindest eine vierte Population von Neuronen im Simulationsmodell angehört, wobei vorzugsweise die vierte Population mit der zweiten Population gegenseitig erregend wirkt und die vierte Population auf die dritte Population erregend wirkt und umgekehrt von der dritten Population mit einer Verzögerung gehemmt wird.

7. Verfahren nach Anspruch 6, **dadurch gekennzeichnet, dass** dem zentralen Mustergenerator zumindest eine fünfte Population von Neuronen im Simulationsmodell angehört, wobei die fünfte Population eine selbst erregende Population ist, wobei die fünfte Population vorzugsweise erregend auf die zweite Population wirkt.

8. Verfahren nach Anspruch 6 oder 7, **dadurch gekennzeichnet, dass** ein simuliertes physiologisches neuronales Signal durch einen Ausgangswert des zentralen Mustergenerators gebildet ist, wobei dieser Ausgangswert vorzugsweise mit einem gemessenen physiologischen neuronalen Signal verglichen wird, um das Simulationsmodell anzupassen.

9. Verfahren nach Anspruch 8, **dadurch gekennzeichnet, dass** dem zentralen Mustergenerator zumindest eine sechste Population von Neuronen im Simulationsmodell angehört, wobei die sechste Population von der vierten Population erregt wird und wobei ein Ausgangswert der sechsten Population als Ausgangswert des zentralen Mustergenerator verwendet wird.

10. Verfahren nach Anspruch 9, **dadurch gekennzeichnet, dass** dem zentralen Mustergenerator zumindest eine siebte Population von Neuronen Simulationsmodell angehört, wobei die siebte Population hemmend auf die sechste Population wirkt.

11. Verfahren nach einem der Ansprüche 2 bis 10, **dadurch gekennzeichnet, dass** die Wechselwirkung zwischen den Populationen durch Moderationsparameter beeinflusst wird, wobei jeder Population ein Moderationsparameter jeweils zugeordnet ist, wobei die Moderationsparameter ihrerseits einer zeitlichen Entwicklung folgen, die von der Erregung der jeweiligen Population abhängig ist.

12. Verfahren zur Ermittlung eines elektrischen Signals zur Behandlung einer neurologischen Dysfunktion eines Patienten,
wobei ein Verfahren zur Simulation von physiologischen neuronalen Signalen nach einem der Ansprüche 1 bis 11 eingesetzt wird,
wobei ausgehend von gemessenen physiologischen neuronalen Signalen des Patienten ein Simulationsmodell zur Nachbildung dieser physiologischen neuronalen Signale angepasst wird,
wobei anhand des angepassten Simulationsmodells ein externes elektrisches Signal simuliert wird und die Parameter des elektrischen Signals derart angepasst werden, dass die nachgebildeten physiologischen neuronalen Signalen einem Signalverlauf ohne Dysfunktion angenähert werden.

13. Verfahren nach Anspruch 12, **dadurch gekennzeichnet, dass** das Verfahren zur Ermittlung eines elektrischen Signals zur Behandlung einer neurologischen Dysfunktion eines Patienten im Bereich des Bewegungsapparats, insbesondere im Bereich des lumbalen Rückenmarks, verwendet wird.

14. System zur Datenverarbeitung, umfassend Mittel zur Ausführung des Verfahrens nach einem der Ansprüche 1 bis 13.

15. Computerprogrammprodukt, umfassend Befehle, die bei der Ausführung des Programms durch einen Computer diesen veranlassen, das Verfahren nach einem der Ansprüche 1 bis 13 auszuführen.
